# FASCICULE DE BREVET EUROPEEN

(11) **EP 1 611 170 B1**
(45) Date de publication et mention de la délivrance du brevet: **06.07.2011**
(21) Numéro de dépôt: 04742489.0
(22) Date de dépôt: 13.04.2004
(51) Int. Cl.: C08F 38/00

(54) **MACROMOLECULES AUTO ASSEMBLEES AUTOUR DE NANOTUBES DE CARBONE, UN PROCEDE POUR LEUR PREPARATION, ET LEURS APPLICATIONS**
MAKROMOLEKÜLAUTOVERSAMMLUNGSPRODUKT UM KOHLENSTOFF-NANORÖHREN, EIN VERFAHREN ZU IHRER HERSTELLUNG UND VERWENDUNG
MACROMOLECULES AUTO-ASSEMBLED AND PHOTOPOLYMERISED AROUND CARBON NANOTUBES A METHOD FOR PRODUCTION AND APPLICATION THEREOF

(30) Priorité: 10.04.2003 FR 0304492
(43) Date de publication de la demande: 04.01.2006
(73) Titulaire: CENTRE NATIONAL DE LA RECHERCHE SCIENTIFIQUE (CNRS), 75794 Paris Cedex 16 (FR); Université Louis Pasteur, 67070 Strasbourg Cedex (FR)
(72) Inventeur: MIOSKOWSKI, Charles, F-67200 STRASBOURG (FR); RICKLING, Stéphane, F-67000 STRASBOURG (FR); SCHULTZ, Patrick, F-67640 FEGERSHEIM (FR)
(74) Mandataire: Peaucelle, Chantal
(86) Numéro de dépôt international: PCT/FR2004/000906
(87) Numéro de publication internationale: WO 2004/092231

(56) Documents cités:
- WO-A-02/44336
- WO-A-99/57564
- WO-A-99/61912
- US-B1- 6 426 134
- PATENT ABSTRACTS OF JAPAN vol. 2003, no. 12, 5 décembre 2003 (2003-12-05) & JP 2004 082663 A (SOGO PHARMACEUTICAL CO LTD), 18 mars 2004 (2004-03-18)
- DATABASE WPI Section Ch, Week 200020 Derwent Publications Ltd., London, GB; Class A85, AN 2000-232848 XP002264347 & JP 2000 053407 A (OSAKA GAS CO LTD) 22 février 2000 (2000-02-22)

## Description

L'invention a pour objet des macromolécules auto assemblées et photopolymérisées autour de nanotubes de carbone, un procédé pour leur préparation, et leurs applications.

Depuis leur découverte au début des années 1990 par Iijima (Nature 354, 56(1991)), les nanotubes et notamment les nanotubes de carbone, ont suscité un intérêt croissant en raison de leurs propriétés physiques, électroniques ou thermiques. La majorité des applications nécessitent un très haut niveau de pureté des nanotubes, et de nombreuses méthodes de purifications ont été décrites, que ce soit par oxydation, par filtration ou encore par chromatographie. Très souvent, après ces processus, les nanotubes sont endommagés (oxydation, rognures aux extrémités) ou voient leur structure graphitique modifiée (fonctionnalisation covalente sur les bouts ou les parois latérales des tubes), ce qui entraîne une altération parfois très importante de leurs propriétés.

On mesure donc l'intérêt d'une méthode de purification efficace et non destructive.

Les travaux des inventeurs dans ce domaine montrent que certains composés peuvent s'auto-organiser autour de nanotubes en formant des anneaux et les protéger ainsi contre toute détérioration au cours de leur manipulation. Avantageusement, ces composés peuvent être détachés des nanotubes et récupérés pour des applications d'intérêts, les nanotubes étant alors obtenus avec un niveau de pureté très élevé.

L'invention a donc pour but de fournir de nouveaux composés utilisables pour la protection des nanotubes.

Elle vise également une méthode de purification de nanotubes mettant en oeuvre ces composés.

Selon encore un autre aspect, l'invention vise les nouvelles structures avec auto-assemblage desdits composés autour des nanotubes et leurs applications, notamment pour la protection et la purification de nanotubes, ou encore la vectorisation de produits actifs.

Les nouvelles structures avec auto-organisation de macromolécules autour des nanotubes sont caractérisées en ce qu'elles sont essentiellement formées d'anneaux de composés lipidiques polymérisés entourant des nanotubes, ces composés polymérisés étant obtenus à partir de composés lipidiques, comportant une ou deux chaînes A liées à un groupe Z :
- A représentant une chaîne, CH₃-(CH₂)ₘ-C≡C-C≡C-(CH₂)ₙ-
- n et m, identiques ou différents, étant des entiers de 1 à 16, et
- Z représentant une tête polaire constituée par un groupe -COOH, -CO-NH-Y, -NH₂ ou N⁺(R)₃, R étant un alkyle de C1 à C4, et Y un radical - (CH₂)₄ -C (R₁) -N (CH₂-COOH) ₂ , avec R₁ représentant H ou un radical COOH dans le cas ou A représente une seule chaîne lipidique, ou un groupe de structure : 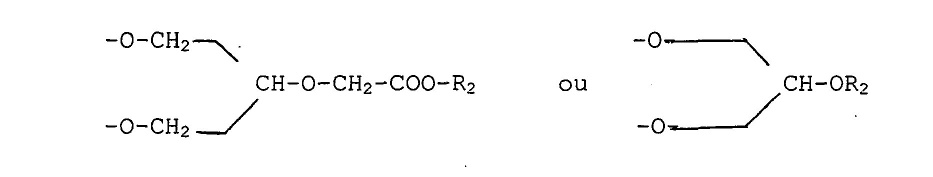
avec R₂ représentant un groupe -COOH, -CO-NH-Y₁, Y₁ étant un radical -(CH₂)₄ -C(R₃)-N(CH₂-COOH)₂, avec R₃ représentant H ou un radical COOH,
ou Z ou R₂ peuvent être également des têtes polaires hydrophiles, neutres, de type sucre ou polysaccharide.

Des composés lipidiques préférés polymérisables sont des lipides amine de formule

CH₃-(CH₂)ₘ -C≡C-C≡C- (CH₂) ₙ-NH₂

ou des ammoniums quaternaires de formule

CH₃-(CH₂)ₘ -C≡C-C≡C-(CH₂)ₙ-N⁺(R)₃

D'autres composés lipidiques préférés polymérisables sont des lipides acides bi-brins, c'est-à-dire avec deux chaînes A fixées à Z.

D'autres composés encore sont des lipides fonctionnalisés par un groupe chélatant, comme l'acide nitrilotriacétique (NTA) ou l'acide iminodiacétique (IDA).

L'invention vise également un procédé d'obtention des structures définies ci-dessus, caractérisé en ce qu'il comprend les étapes consistant à
- mettre en contact des nanotubes bruts avec une solution de lipides de manière à former une suspension stable ;
- polymériser les lipides qui se sont auto-arrangés autour des nanotubes ;
- récupérer les nanotubes enrobés par des anneaux constitués par les lipides polymérisés.

Avantageusement, les nanotubes bruts sont soniqués dans la solution de lipides.

Dans le procédé de l'invention, ces lipides sont en solution dans un milieu aqueux tamponné contenant avantageusement un détergent. On citera comme détergent, le sodium dodécyl sulfate (SDS).

Après la sonication, le détergent est éliminé par dialyse.

La suspension de nanotubes dans le tampon aqueux est soumise à un traitement pour polymériser les lipides.

On a recours à une irradiation sous ultra-violet.

Dans un mode de mise en oeuvre de l'invention, les structures obtenues sont soumises à un traitement afin de séparer les nanotubes entourés d'anneaux lipidiques polymérisés de toutes les impuretés contenues dans le brut de synthèse des nanotubes.

Cette étape est réalisée, par exemple, par chromatographie par exclusion de taille.

Des phases stationnaires constituées par de la silice à porosité contrôlée, tel que le produit commercialisé sous la dénomination CPG (pour "controlled pore glass") par Millipore Corp., se sont révélées satisfaisantes. Une ou plusieurs étapes de purifications peuvent être réalisées et la porosité avantageusement modifiée selon l'étape de purification. On obtient ainsi des nanotubes de grande pureté.

Il est également possible de retirer les anneaux en appliquant un champ électrique, par exemple dans un dispositif d'électrophorèse.

Les nanotubes entourés de macromolécules polymérisées ainsi obtenus présentent des propriétés avantageuses dans de nombreuses applications.

Les nanotubes entourés des hémi-micelles photopolymérisées permettent d'effectuer, notamment, un raccourcissement contrôlé des tubes par sonication.

On sait que les nanotubes monofeuillets sont sensibles à la sonication forte et qu'une exposition prolongée à des ultrasons provoque une forte dégradation des tubes, essentiellement par un phénomène de rognure des nanotubes par leurs extrémités.

De même, on a pu noter que les parois latérales des nanotubes sont fortement endommagées après une sonication intense, ce qui perturbe la structure graphitique des nanotubes et altère leurs propriétés électroniques.

Il s'avère que les nanotubes mono parois de l'invention, qui sont entourés de macromolécules polymérisées, permettent de contrôler leur raccourcissement.

Les inventeurs ont en effet constaté qu'en soumettant des échantillons de nanotubes mono parois, recouverts de lipides polymérisés, tel que décrit ci-dessus, à un traitement de sonication, il est possible de couper les nanotubes et d'atteindre, par exemple, des tailles moyennes de l'ordre de 400 nm après 2 heures de sonication.

Comme illustré dans les exemples, on peut ainsi obtenir des nanotubes mono parois coupés à des tailles voisines sous forme de tubes isolés ou en petits fagots, ce qui montre que la coupure ne s'est pas faite par une rognure aux extrémités, mais plutôt par cassure des nanotubes en deux.

Le lipide polymérisé à la surface des nanotubes sert donc de protection pour les nanotubes.

Les espèces polymériques de l'invention en forme d'anneaux qui ont été détachés des nanotubes de carbone par électrophorèse constituent de nouveaux vecteurs de molécules hydrophobes ou de protéines membranaires, car l'intérieur de ces anneaux est de structure bicouche membranaire.

Comme indiqué ci-dessus, les macromolécules formées de lipides polymérisés à la surface des nanotubes sont hydrophobes dans leur partie interne et hydrophiles dans leur partie externe.

Ils constituent donc des vecteurs d'intérêt pour des molécules hydrophobes dans des milieux aqueux.

L'invention vise donc l'application des anneaux polymériques détachés des nanotubes comme vecteurs de produits hydrophobes.

Les produits hydrophobes auront tendance, lorsqu'on les met en contact avec lesdites macromolécules polymérisées, à se placer à l'intérieur de la poche hydrophobe présentée par les lipides polymérisés. Ces derniers sont solubles en milieu aqueux en raison de leur partie hydrophile extérieure et l'ensemble lipide/produit hydrophobe sera alors également soluble.

Cette application utilise les nanotubes pour la fabrication de transporteurs de molécules non hydrosolubles en particulier des médicaments et des protéines hydrophobes.

En utilisant des lipides de taille appropriée, il est possible de disposer de recouvrements mimant la membrane cellulaire, les macromolécules polymérisées autour des nanotubes pouvant être apparentées à des bi-couches lipidiques.

Avec des lipides dont la taille est de l'ordre de grandeur de celle des lipides d'une membrane cellulaire, et des nanotubes de diamètre approprié, l'invention fournit des structures dont les anneaux de recouvrement permettent de solubiliser des protéines membranaires. En mettant en contact, en milieu aqueux, des protéines membranaires avec des anneaux lipidiques polymérisés selon l'invention, la partie hydrophobe des protéines membranaires entre en contact avec l'intérieur des anneaux. Le complexe macromolécule/ protéine est alors soluble en phase aqueuse, ce qui permet de solubiliser des protéines membranaires sans avoir recours à des détergents qui risquent de les endommager ou de les dénaturer.

Selon un autre aspect de grand intérêt, l'invention vise l'application des nanotubes mono et multi feuillets comme moteurs moléculaires.

Les nanotubes mis en oeuvre comportent au plus quelques anneaux de lipide polymérisé, de préférence un seul anneau, et sont soumis à un champ électrique alternatif ou non de manière à déplacer le ou les anneaux le long du nanotube.

Les nouvelles structures de l'invention avec auto-organisation de macromolécules autour des nanotubes sont également utilisables pour la vectorisation de produits en général, permettant leur délivrance spécifique ou non spécifique. Elles présentent ainsi un grand intérêt dans divers domaines, notamment en pharmacie pour l'encapsulation de principes actifs de médicaments, ou en cosmétique et parfumerie, pour la vectorisation de fragrances, huiles essentielles et autres, ou pour l'encapsulation de divers produits actifs comme les phéronomes. Ces structures sont donc avantageusement utilisables dans les applications habituelles des liposomes.

D'autres caractéristiques et avantages de l'invention seront donnés dans les exemples qui suivent et en se référant aux figures 1 à 3, qui représentent respectivement des photos au microscope électronique à transmission de structures et de nanotubes selon l'invention,
- les figures 1a et 1b, correspondant respectivement à un échantillon brut de nanotubes mono parois et à un échantillon après une 2^{ème} étape de purification ;
- les figures 2a et 2b, correspondant respectivement à un échantillon brut de nanotubes multi feuillets et à un échantillon après une 2^{ème} étape de purification ; et
- la figure 3 a des nanotubes mono parois coupés après sonication forte d'une heure.

### Exemple 1 : procédé d'obtention de structures selon l'invention

Un échantillon de nanotubes bruts mono ou multi feuillets, quel que soit le procédé de synthèse, est soniqué pendant 5 min dans une solution de lipide (11,8) NTA (lipide à fonctionnalité nitrilotriacétique), à une concentration de 1mg/mL de tampon Tris (pH8) contenant 1% de SDS. Après sonication, les nanotubes sont sous forme de suspension stable dans le tampon aqueux. Le détergent est éliminé par dialyse contre du Tris sans SDS, pendant 48 h, en changeant le Tris toutes les 12 h. Une fois la dialyse terminée, on polymérise le lipide qui s'est auto-arrangé autour des nanotubes en irradiant l'échantillon sous UV (lambda = 254 nm) pendant 1 h, et on récupère les structures formées.

### Exemple 2 : procédé de purification de nanotubes

On utilise la technique de chromatographie par exclusion de taille avec comme phase stationnaire du CPG, commercialisé par Millipore Corp. (Lincoln Park, NJ, EUA).

Une première étape de purification est réalisée avec du CPG 3 000 A ayant une taille de pores moyenne de 300nm. Cette phase stationnaire est placée dans une colonne de 14 cm x 0,7 cm, et conditionnée avec une solution aqueuse de SDS à une concentration de 0,25%.

0,5mL de suspension de nanotubes irradiés (1mg/mL) monoparois ou multifeuillets, et déposé sur la colonne et est élué avec du SDS aqueux à une concentration de 0,25%. Le flux de l'éluant est réglé à environ 10mL/h. Après un volume mort de 4mL, on collecte 6 fractions de 2mL que l'on observe par microscopie électronique à transmission (TEM).

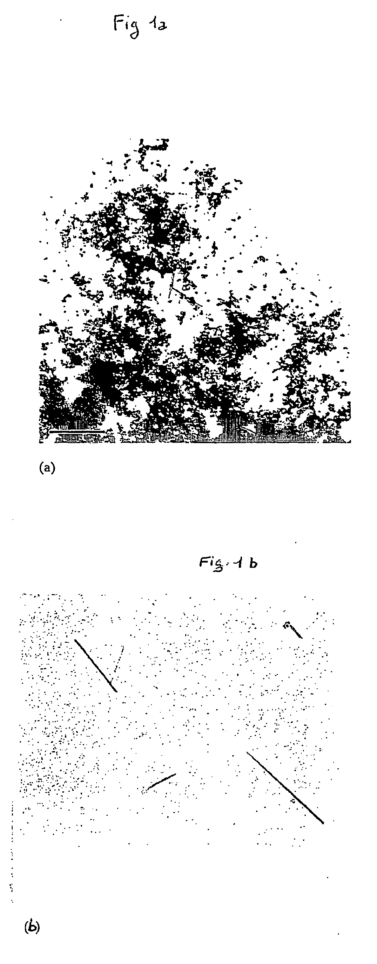

Pour les nanotubes multifeuillets, on observe un maximum de tubes dans la première fraction avec quelques impuretés (Fig.1). Les fractions suivantes contiennent essentiellement du carbone amorphe et d'autres impuretés, et quelques rares nanotubes. La première fraction est alors engagée dans une deuxième étape de purification en déposant 0,5mL sur une colonne de 14 cm x 0,7cm contenant du CPG 1400 A (taille moyenne des cavités : 140 nm). Le même éluant est utilisé, et après un volume mort de 6mL, on récupère 6 fractions de 0,5mL, le flux d'éluant étant réglé à environ 10mL/h. Des observations TEM ont montrés que la deuxième fraction contenait des nanotubes multifeuillets purs, pratiquement dépourvus d'impuretés.

Le même procédé est utilisé pour la purification des nanotubes monoparois. Après la première étape de purification sur la colonne CPG 3000 A, on collecte des fractions de 2mL après un volume mort de 4mL.

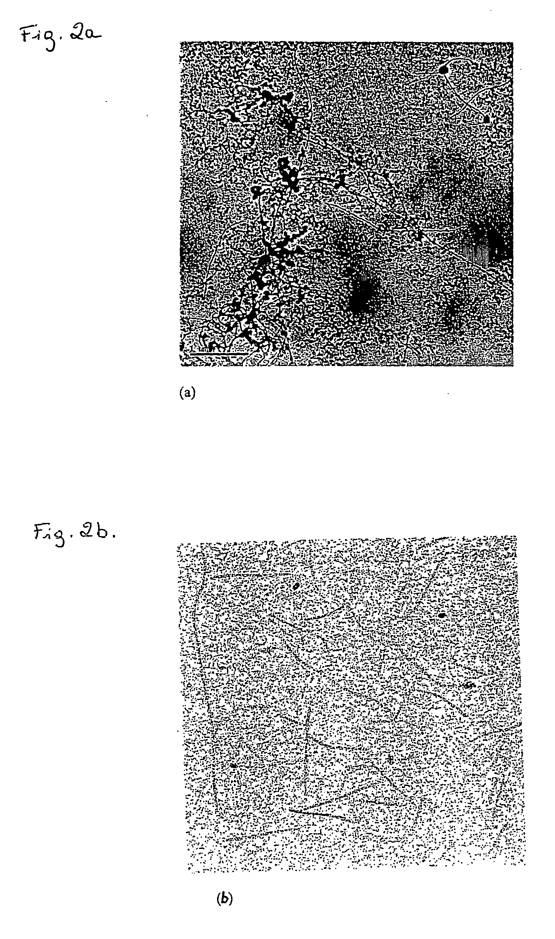

L'observation au microscope montre que la première des 6 fractions contient les nanotubes les plus purs (Fig.2).

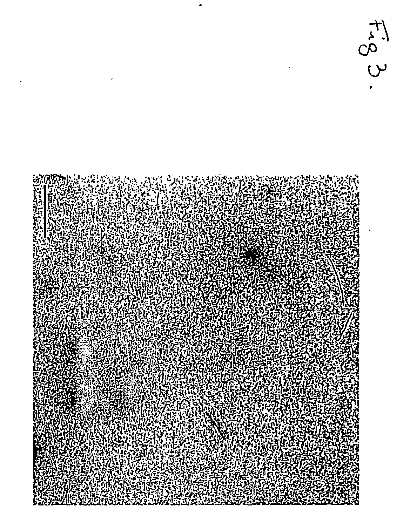

Les fractions suivantes contiennent très peu de nanotubes et une grande majorités d'impuretés. La première fraction est réengagée dans un nouveau cycle de purification, en utilisant une nouvelle colonne CPG 3000 A (volume mort : 2 mL ; fractions de 0,5 mL). On récupère 6 fractions. L'observation au microscope montre que les fractions 4 et 5 contiennent des nanotubes monoparois avec une pureté supérieure à 95% (Fig.3).

Les échantillons de nanotubes purifiés sont assemblées et centrifugés jusqu'à l'obtention d'un dépôt noir et d'un surnageant translucide. On enlève le surnageant et on lave les nanotubes déposés avec de l'eau pure afin d'enlever le reste de détergent.

Après 3 min de sonication, on centrifuge à nouveau l'échantillon, on enlève le surnageant, et on recommence les étapes de lavage, sonication et centrifugation à 3 reprises.

Le solide déposé après la dernière centrifugation est lyophilisé pour donner un échantillon sec de nanotubes propres recouverts de lipides polymérisé.

### Exemple 3 : Procédé d'obtention des nanotubes dépourvus de polymère lipidique

Les nanotubes obtenus dans l'exemple 2 sont portés à une température supérieure à 90°C pendant environ 14 h dans du tampon Tris, ce qui conduit à la destruction des lipides polymérisés. Un lavage avec un solvant organique, comme par exemple le méthanol ou l'éthanol, permet d'enlever le reste de polymère.

### Exemple 4 Procédé d'obtention d'anneaux lipidiques polymérisées utilisables en tant que vecteurs de molécules ou de protéines hydrophobes

Les nanotubes entourés de macromolécules polymériques sont soumis à une électrophorèse sur gel d'agarose en utilisant un électrolyte contenant un tampon Tris + glycine avec 0,1% de SDS. Ainsi, les anneaux moléculaires peuvent être détachés des nanotubes de carbone et récupérés afin d'être utilisés dans d'autres applications.

### Exemple 5 : Préparation d'un nanotube avec anneaux de macromolécules polymérisées utilisables comme moteurs moléculaires

On mélange une faible proportion de lipide photopolymérisable avec un autre lipide non photopolymérisable choisi parmi des lipides ne formant pas de micelles mixtes, en solution aqueuse, avec le lipide photopolymérisable. Un lipide non photopolymérisable approprié contient une extrémité faiblement perfluorée, qui ne formera des micelles qu'avec des lipides du même type et ne formera pas de micelles mixtes avec des lipides entièrement hydrogénés.

On procède comme indiqué ci-dessus. On obtient des nanotubes avec deux types de micelles et donc deux types d'anneaux, un seul étant photopolymérisable. Etant donné que les lipides photopolymérisables sont pris en large défaut par rapport à l'autre type de lipides, il ne se forme que très peu d'arrangements polymérisables sur les nanotubes. Après irradiation par UV, les produits sont lavés avec un solvant organique, comme le méthanol ou l'éthanol, afin de retirer tout élément nonpolymérisé autour des nanotubes.

Il ne reste alors que quelques anneaux, de préférence un seul anneau, sur le nanotubes qui sont utilisables dans l'application comme nanomoteurs.

### Exemple 6 Synthèse des composés lipidiques utilisés pour la fabrication des structures de l'invention

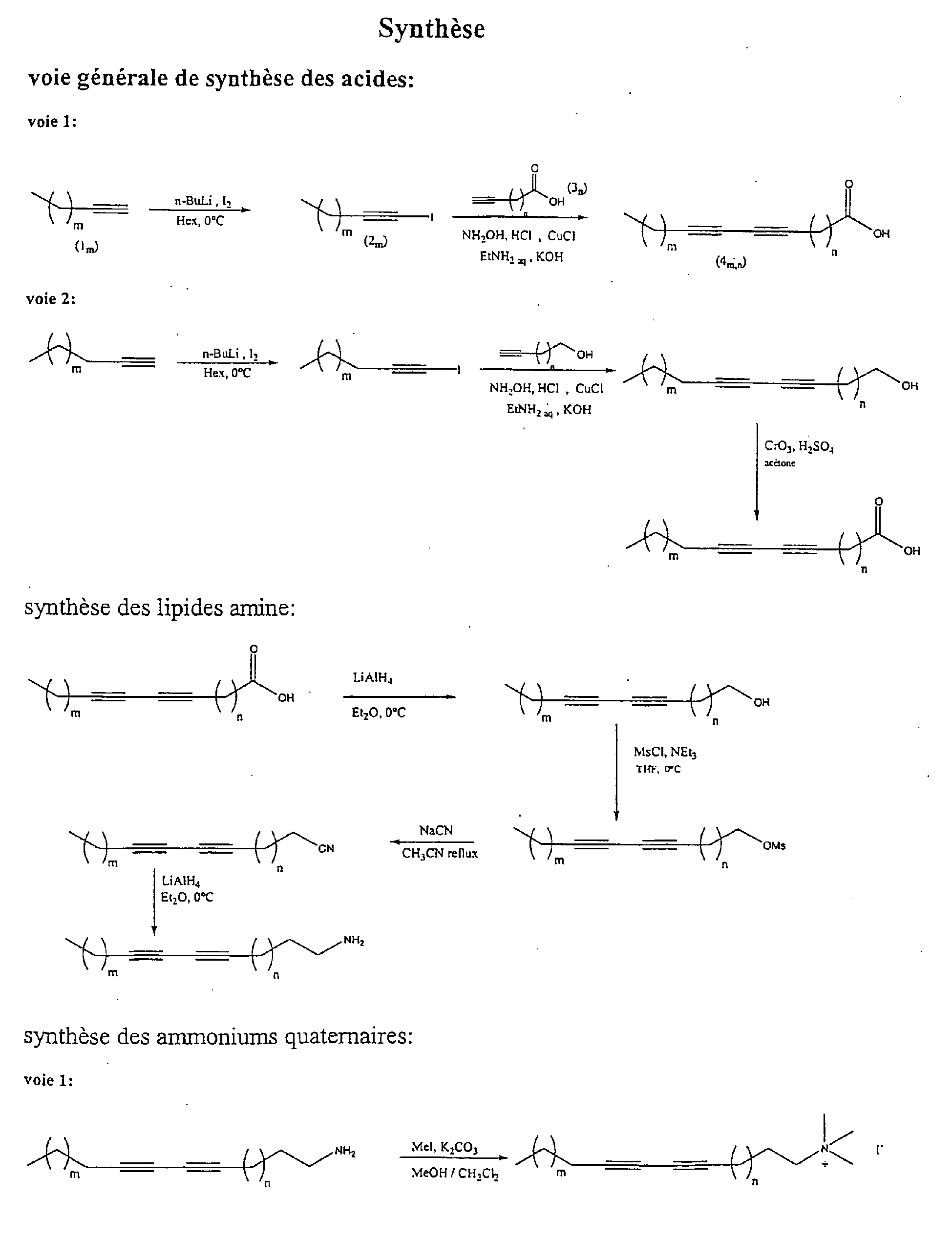

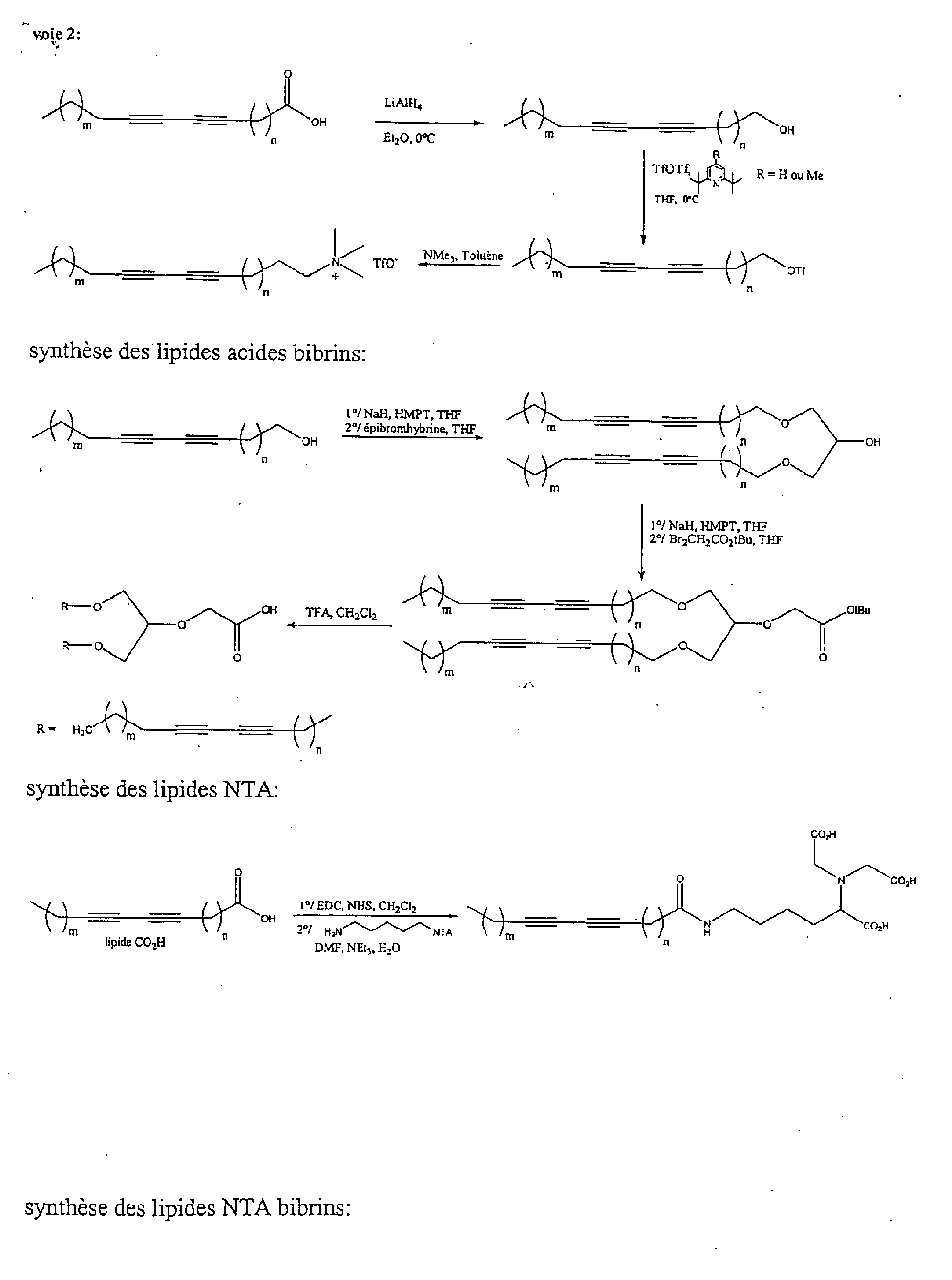

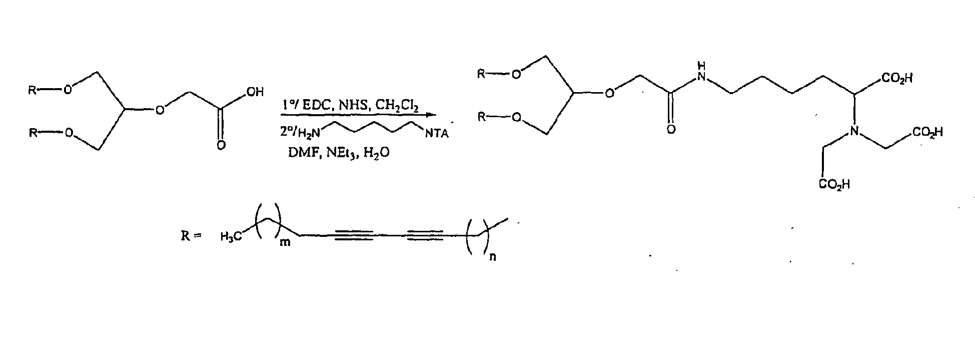

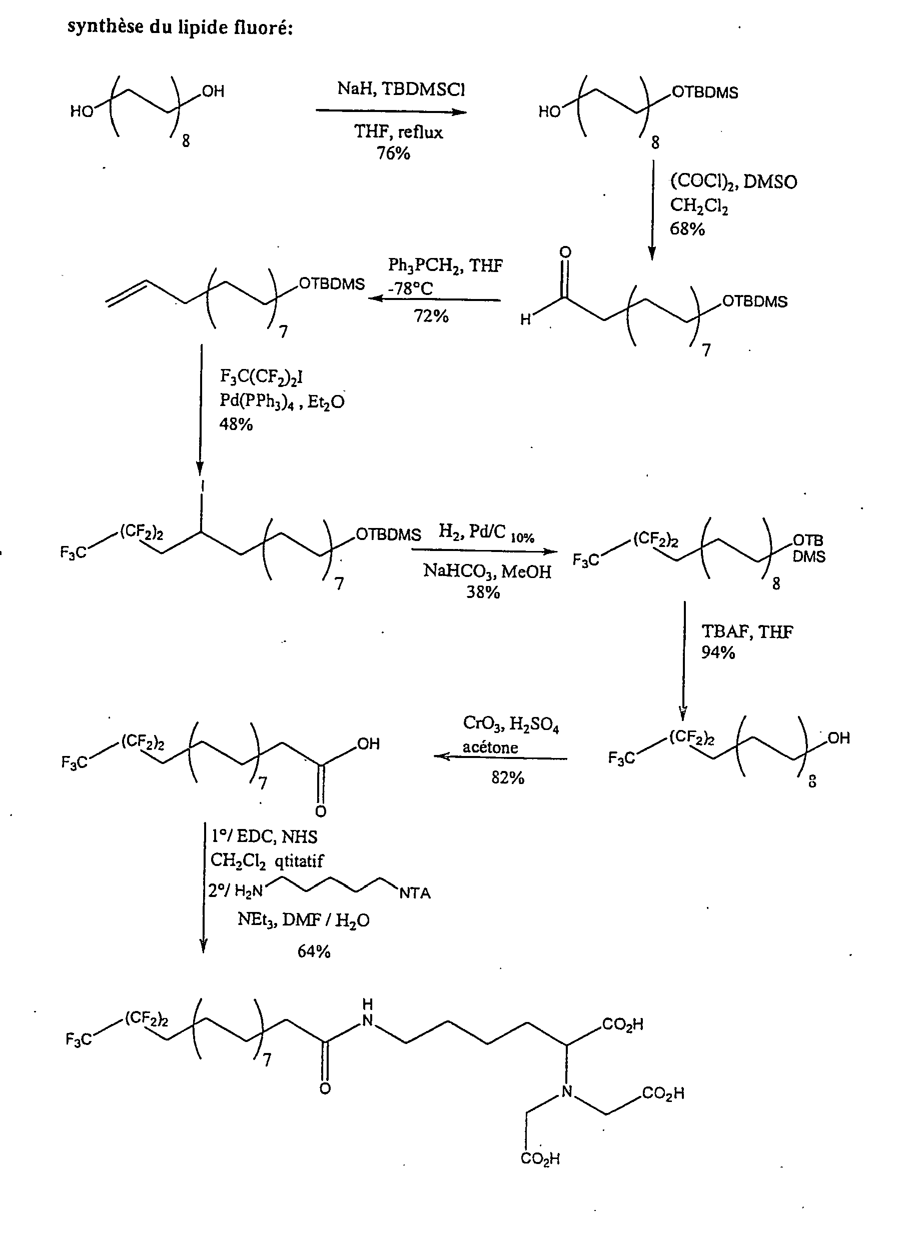

## Revendications

1. Structures avec auto-organisation de macromolécules autour des nanotubes, **caractérisées en ce qu'**elles sont essentiellement formées d'anneaux de composés lipidiques polymérisés entourant des nanotubes, ces composés polymérisés étant obtenus à partir de composés lipidiques, comportant une ou deux chaînes A liées à un groupe Z :
- A représentant une chaîne, CH₃-(CH₂)ₘ-C≡C-C≡C-(CH₂)ₙ-, n et m, identiques ou différents, étant des entiers de 1 à 16, et
- Z représentant une tête polaire constituée par un groupe -COOH, -CO-NH-Y, -NH₂ ou N⁺(R)₃, R étant un alkyle de C1 à C4, et Y un radical -(CH₂)₄-C(R₁)-N(CH₂-COOH)₂, avec R₁ représentant H ou un radical COOH dans le cas ou A représente une seule chaîne lipidique, ou un groupe de structure : 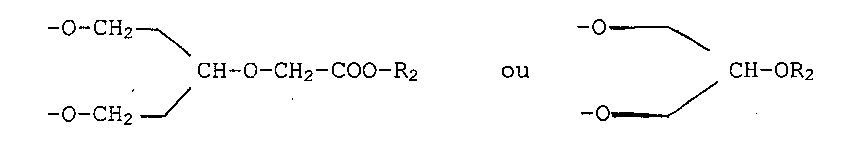
avec R₂ représentant un groupe -COOH, -CO-NH-Y₁, Y₁ étant un radical -(CH₂)₄ -C(R₃)-N(CH₂-COOH)₂, avec R₃ représentant H ou un radical COOH,
ou Z ou R₂ peuvent être également des têtes polaires hydrophiles, neutres, de type sucre ou polysaccharide.
Z ou R₂ pouvant être également des têtes polaires hydrophiles neutres de type sucre ou polysaccharide.

2. Structures selon la revendication 1, **caractérisées en ce que** les composés lipidiques à polymériser sont des lipides amine de formule
CH₃-(CH₂)ₘ -C≡C-C≡C- (CH₂) ₙ-NH₂

3. Structures selon la revendication 1, **caractérisées en ce que** les composés lipidiques à polymériser sont des ammoniums quaternaires de formule CH₃-(CH₂)ₘ -C≡C-C≡C- (CH₂) ₙ-N⁺ (R) ₃.

4. Structures selon la revendication 1, **caractérisées en ce que** les composés lipidiques à polymériser sont des lipides acides avec deux chaînes A fixées à Z.

5. Structures selon l'une quelconque des revendications 2 à 4, **caractérisées en ce que** les composés lipidiques à polymériser sont fonctionnalisés par un groupe chélatant.

6. Structures selon la revendication 1, **caractérisées en ce que** les composés lipidiques à polymériser sont fonctionnalisés par une tête hydrophile neutre de type sucre ou polysaccharide.

7. Procédé d'obtention des structures selon l'une quelconque des revendications 1 à 6, **caractérisé en ce qu'**il comprend les étapes consistant à :
- mettre en contact des nanotubes bruts avec une solution de lipides de manière à former une suspension stable ;
- polymériser les lipides qui se sont auto-arrangés autour des nanotubes ;
- récupérer les nanotubes enrobés par des anneaux constitués par les lipides polymérisés.

8. Procédé selon la revendication 7, **caractérisé en ce que** les nanotubes bruts sont soniqués dans une solution de lipides dans un milieu aqueux tamponné contenant avantageusement un détergent, ce dernier étant éliminé ensuite par dialyse, puis la suspension de nanotubes dans le tampon aqueux est soumise à un traitement pour polymériser les lipides.

9. Procédé de purification de nanotubes, **caractérisé en ce qu'**on soumet les structures selon l'une quelconque des revendications 1 à 6 à un traitement afin d'éliminer les anneaux de composés lipidiques polymérisés entourant les nanotubes.

10. Procédé selon la revendication 9, **caractérisé en ce qu'**on soumet lesdites structures à une chromatographie par exclusion de taille.

11. Procédé selon la revendication 9, **caractérisé en ce qu'**on applique un champ électrique pour retirer les anneaux.

12. Procédé selon la revendication 9, **caractérisé en ce qu'**on soumet lesdites structures à un chauffage dans du tampon Tris à une température supérieure à 90°C pendant 14 heures environ pour enlever le polymère et restaurer les nanotubes dénudés.

13. Application des structures selon l'une quelconque des revendications 1 à 6, pour protéger les nanotubes et effectuer si souhaité un raccourcissement contrôlé de ces nanotubes.

14. Application des structures selon l'une quelconque des revendications 1 à 6, comme vecteurs de molécules hydrophobes ou de protéines membranaires.

15. Application des structures selon l'une quelconque des revendications 1 à 6, comme moteurs moléculaires.

16. Application des structures selon l'une quelconque des revendications 1 à 6, à la vectorisation de produits notamment dans le domaine pharmaceutique ou cosmétique ou en parfumerie.

## Claims

1. Structures with self-organization of macromolecules around nanotubes, **characterized in that** they are essentially formed from rings of polymerized lipid compounds surrounding the nanotubes, these polymerized compounds being obtained from lipid compounds comprising one or two chains A linked to a group Z:
- A representing a CH₃-(CH₂)ₘ-C≡C-C≡C-(CH₂)ₙ-, n and m, which are the same or different, being integers from 1 to 16; and
- Z representing a polar head formed by a -COOH, -CO-NH-Y, -NH₂ or N⁺(R)₃ group, R being a C1 to C4 alkyl and Y being a -(CH₂)₄ -C(R₁) -N(CH₂-COOH)₂ radical, with R₁ representing H or a COOH radical if A represents a single lipid chain, or a group of structure: 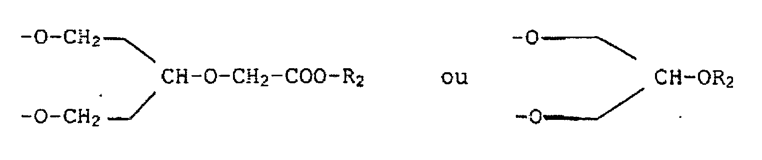
where R₂ represents a -COOH or -CO-NH-Y₁ group, Y₁ being a -(CH₂)₄ -C(R₃)-N(CH₂-COOH)₂ radical, with R₃ representing H or a COOH radical;
or Z or R₂ may also be hydrophilic or neutral polar heads of the sugar or polysaccharide type,
or Z or R₂ may also be hydrophilic or neutral polar heads, of the sugar or polysaccharide type.

2. The structures according to claim 1, **characterized in that** the lipid compounds to be polymerized are amine lipids of formula:
CH₃ -(CH₂)ₘ -C=C- C=C- (CH₂)ₙ-NH₂

3. The structures according to claim 1, **characterized in that** the lipid compounds to be polymerized are quaternary ammoniums of formula: CH₃-(CH₂)ₘ -C≡C -C=C- (CH₂)ₙ-N⁺(R)₃.

4. The structures according to claim 1, **characterized in that** the lipid compounds to be polymerized are acid lipids with two chains A attached to Z.

5. The structures according to anyone of claims 2 to 4, **characterized in that** the lipid compounds to be polymerized are functionalized by a chelating group.

6. The structures according to claim 1, **characterized in that** the lipid compounds to be polymerized are functionalized by a neutral hydrophilic head of the sugar or polysaccharide type.

7. A method of obtaining the structures according to anyone of claims 1 to 6, **characterized in that** it comprises the steps consisting in:
- bringing the raw nanotubes into contact with a solution of lipids so as to form a stable suspension;
- polymerizing the lipids, which are self-organized around the nanotubes; and recovering the nanotubes coated with rings formed by the polymerized lipids.

8. The method according to claim 7, **characterized in that** the raw nanotubes are sonicated in a lipid solution in a buffered aqueous medium advantageously containing a detergent, the latter being subsequently removed by dialysis, and then the suspension of nanotubes in the aqueous buffer is subjected to a treatment for polymerizing the lipids.

9. A method of purifying nanotubes, **characterized in that** the structures according to anyone of claims 1 to 6 are subjected to a treatment so as to remove the rings of polymerized lipid compounds around the nanotubes.

10. The method according to claim 9, **characterized in that** said structures are subjected to size exclusion chromatography.

11. The method according to claim 9, **characterized in that** an electric field is applied in order to remove the rings.

12. The method according to claim 9, **characterized in that** said structures are heated in a Tris buffer at a temperature above 90°C. for about 14 hours in order to remove the polymer and restore the stripped nanotubes.

13. Application of the structures according to anyone of claims 1 to 6, for protecting the nanotubes and, if required, for shortening these nanotubes in a controlled manner.

14. Application of the structures according to anyone of claims 1 to 6, as vectors for hydrophobic molecules or membrane proteins.

15. Application of the structures according to anyone of claims 1 to 6, as molecular motors.

16. Application of the structures according to anyone of claims 1 to 6, to the vectorization of products, especially in the pharmaceutical or cosmetic or perfumery field.

## Patentansprüche

1. Strukturen mit Selbstorganisation von Makromolekülen um Nanoröhrchen herum, **dadurch gekennzeichnet, dass** sie im Wesentlichen aus Ringen von polymerisierten Lipidverbindungen bestehen, die Nanoröhrchen umgeben, wobei diese polymerisierten Verbindungen ausgehend von Lipidverbindungen erhalten werden, die eine oder zwei Ketten A umfassen, welche an eine Gruppe Z gebunden sind:
- wobei A für eine Kette CH₃-(CH₂)ₘ-C≡C-C≡C-(CH₂)ₙ- steht, wobei n und m gleich oder verschieden sind und ganze Zahlen von 1 bis 16 sind; und
- wobei Z für einen polaren Kopf steht, der von einer Gruppe -COOH, -CO-NH-Y, -NH₂ oder N⁺(R)₃, wobei R ein C₁- bis C₄-Alkyl ist und Y ein Rest -(CH₂)₄-C(R₁)-N(CH₂-COOH)₂ ist, wobei R₁ für H oder einen Rest COOH steht, gebildet wird, wenn A für eine einzige Lipidkette steht, oder von einer Gruppe der Struktur 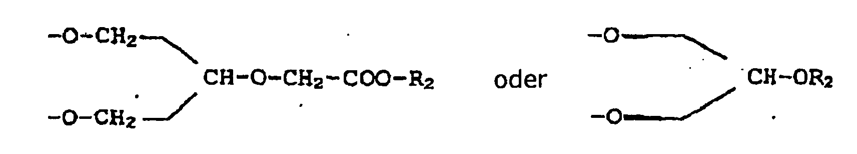
gebildet wird, wobei R₂ für eine Gruppe -COOH, -CO-NH-Y₁ steht, wobei Y₁ ein Rest -(CH₂)₄-C(R₃)-N(CH₂-COOH)₂ ist, wobei R₃ für H oder einen Rest COOH steht;
oder Z oder R₂ auch neutrale hydrophile polare Köpfe des Zucker- oder Polysaccharid-Typs sein können;
wobei Z oder R₂ auch neutrale hydrophile polare Köpfe des Zucker- oder Polysaccharid-Typs sein können.

2. Strukturen gemäß Anspruch 1, **dadurch gekennzeichnet, dass** die zu polymerisierenden Lipidverbindungen Aminlipide der Formel
CH₃-(CH₂)ₘ-C≡C-C≡C-(CH₂)ₙ-NH₂
sind.

3. Strukturen gemäß Anspruch 1, **dadurch gekennzeichnet, dass** die zu polymerisierenden Lipidverbindungen quartäre Ammoniumverbindungen der Formel CH₃-(CH₂)ₘ-C≡C-C≡C-(CH₂)ₙ-N⁺(R)₃ sind.

4. Strukturen gemäß Anspruch 1, **dadurch gekennzeichnet, dass** die zu polymerisierenden Lipidverbindungen saure Lipide mit zwei Ketten A, die an Z gebunden sind, sind.

5. Strukturen gemäß einem der Ansprüche 2 bis 4, **dadurch gekennzeichnet, dass** die zu polymerisierenden Lipidverbindungen mit einer chelatisierenden Gruppe funktionalisiert sind.

6. Strukturen gemäß Anspruch 1, **dadurch gekennzeichnet, dass** die zu polymerisierenden Lipidverbindungen mit einem neutralen hydrophilen Kopf des Zucker- oder Polysaccharid-Typs funktionalisiert sind.

7. Verfahren zur Gewinnung von Strukturen gemäß einem der Ansprüche 1 bis 6, **dadurch gekennzeichnet, dass** es die folgenden Schritte umfasst:
- In-Kontakt-Bringen von rohen Nanoröhrchen mit einer Lösung von Lipiden, so dass eine stabile Suspension entsteht;
Polymerisieren der Lipide, die sich durch Selbstorganisation um die Nanoröhrchen herum angeordnet haben;
Gewinnen der Nanoröhrchen, die von Ringen umhüllt sind, welche aus den polymerisierten Lipiden bestehen.

8. Verfahren gemäß Anspruch 7, **dadurch gekennzeichnet, dass** die rohen Nanoröhrchen einer Ultraschallbehandlung in einer Lösung von Lipiden in einem gepufferten wässrigen Medium, das vorteilhafterweise ein Tensid enthält, unterzogen werden, wobei das Tensid anschließend durch Dialyse entfernt wird, und dann die Suspension von Nanoröhrchen in dem wässrigen Puffer einer Behandlung zum Polymerisieren der Lipide unterzogen wird.

9. Verfahren zur Reinigung von Nanoröhrchen, **dadurch gekennzeichnet, dass** man die Strukturen gemäß einem der Ansprüche 1 bis 6 einer Behandlung unterzieht, um die Ringe von polymerisierten Lipidverbindungen, die die Nanoröhrchen umgeben, zu entfernen.

10. Verfahren gemäß Anspruch 9, **dadurch gekennzeichnet, dass** man die Strukturen einer Größenausschlusschromatographie unterzieht.

11. Verfahren gemäß Anspruch 9, **dadurch gekennzeichnet, dass** man ein elektrisches Feld anlegt, um die Ringe abzuziehen.

12. Verfahren gemäß Anspruch 9, **dadurch gekennzeichnet, dass** man die Strukturen ungefähr 14 Stunden lang in einem Tris-Puffer bei einer Temperatur von über 90°C erhitzt, um das Polymer zu entfernen und die freigelegten Nanoröhrchen wiederherzustellen.

13. Anwendung der Strukturen gemäß einem der Ansprüche 1 bis 6, um die Nanoröhrchen zu schützen und gegebenenfalls eine kontrollierte Verkürzung dieser Nanoröhrchen durchzuführen.

14. Anwendung der Strukturen gemäß einem der Ansprüche 1 bis 6 als Vektoren von hydrophoben Molekülen oder von Membranproteinen.

15. Anwendung der Strukturen gemäß einem der Ansprüche 1 bis 6 als molekulare Motoren.

16. Anwendung der Strukturen gemäß einem der Ansprüche 1 bis 6 als Träger für Produkte, insbesondere im pharmazeutischen oder kosmetischen Bereich oder in der Parfümerie.
